# EUROPÄISCHE PATENTSCHRIFT

(11) **EP 2 779 966 B1**
(45) Veröffentlichungstag und Bekanntmachung des Hinweises auf die Patenterteilung: **06.01.2016**
(21) Anmeldenummer: 12787738.9
(22) Anmeldetag: 16.11.2012
(51) Int. Cl.: A61F 5/56, A47G 9/10

(54) **KOPFUNTERLAGE ZUM ABSTELLEN VON SCHNARCHEN**
ANTI-SNORING HEAD SUPPORT
SUPPORT DE TÊTE ANTI-RONFLEMENT

(30) Priorität: 16.11.2011 DE 102011118614; 27.03.2012 DE 102012006024; 20.04.2012 US 201261636160 P; 20.04.2012 US 201261636168 P
(43) Veröffentlichungstag der Anmeldung: 24.09.2014
(73) Patentinhaber: Nitetronic Holding Limited, Grand Cayman KY1- 1112 (KY)
(72) Erfinder: HERRNSDORF, Johannes, 58313 Herdecke (DE)
(74) Vertreter: Banse & Steglich
(86) Internationale Anmeldenummer: PCT/EP2012/072923
(87) Internationale Veröffentlichungsnummer: WO 2013/072505

(56) Entgegenhaltungen:
- DE-U1- 9 100 663
- US-A1- 2003 221 261
- US-A1- 2004 177 449
- US-A1- 2009 094 750

## Beschreibung

### Technisches Gebiet

Die vorliegende Erfindung betrifft Kopfunterlagen, insbesondere Kissen. Weiterhin betrifft die vorliegende Erfindung Maßnahmen zum Reduzieren eines Schnarchens einer auf einer Kopfunterlage ruhenden Person.

### Stand der Technik

Ein hoher Anteil von Personen schnarcht während des Schlafens. Schnarchen reduziert die Sauerstoffaufnahme des Körpers und gilt heutzutage als Auslöser für verschiedene Krankheiten. Eine schlafende Person jedoch nimmt das eigene Schnarchen nicht wahr, so dass ein Abstellen des Schnarchens nicht ohne weiteres von der Person selbst herbeigeführt werden kann.

Aus dem Stand der Technik sind vielerlei Ansätze bekannt, um das Schnarchen bei einer schlafenden Person abzustellen. Vorrichtungen, die die schlafende Person beim Auftreten eines Schnarchens bewegen oder die Person zu einer selbstständigen Lageänderung anregen, werden hierbei jedoch im Gegensatz zu Hilfsmitteln, die unmittelbar auf den Körper aufgesetzt oder in den Körper eingesetzt werden, bevorzugt, da sie von den betreffenden Personen besser toleriert werden.

So ist aus der Druckschrift DE 20 121 693 U1 eine Vorrichtung zur Schnarchverhinderung mit einer über eine Steuereinrichtung ansteuerbaren Stelleinrichtung für eine Kopfauflage einer schlafenden Person bekannt, wobei die Kopfauflage bei Auftreten eines Schnarchens zu Schwingungen angeregt wird, die auf den Kopf der schlafenden Person übertragen werden, so dass die schlafende Person das Schnarchen einstellt.

Aus der Druckschrift DE 101 28 095 C2 ist ein Kopfkissen zur Vorbeugung gegen das Auftreten eines Schnarchens bekannt, wobei mindestens eine mit einem Medium gefüllte Druckkammer kontinuierliche, stufenlos regelbare Intervallbewegungen des Kissens über eine Volumenänderung erzeugt, wobei im Kissen selbst keine schädlichen elektrischen Felder entstehen.

Um ein Schnarchen einer auf einer Kopfunterlage ruhenden Person durch eine geeignete Lageänderung des Kopfes abstellen zu können, ist es notwendig, die Position des Kopfes auf der Kopfunterlage mit einer hinreichenden Genauigkeit zu detektieren, so dass der Kopf gezielt bewegt werden kann.

Aus der Druckschrift DE 199 30 818 C1 ist eine Vorrichtung zum Verhindern des Schnarchens bekannt, die ein Kopfkissen, einen Schallsensor zum Aufnehmen von Schnarchgeräuschen und eine durch die Schnarchgeräusche aktivierbare Kontrolleinheit umfasst. Die Kontrolleinheit steuert eine Lageänderung des Kopfes einer schlafenden Person durch Bewegung des Kopfkissens. Dazu wird in dem Kopfkissen ein in Kammern unterteiltes Luftkissen vorgesehen, wobei der Luftdruck der Kammern durch die Kontrolleinheit in Verbindung mit mindestens einer Luftdruckquelle und einer Luftdruckreduziereinheit steuerbar ist. Die Lage des Kopfes des Schläfers kann über die aus der Gewichtskraft des Kopfes resultierende Druckerhöhung in den von dem Kopf belegten Kammern erfasst werden.

Das Vorsehen von Drucksensoren zum Ermitteln des Luftdrucks in den Kammern des Luftkissens ist jedoch aufwändig und es ist daher wünschenswert, eine vereinfachte Detektion der Lage des Kopfes auf der Kopfunterlage zur Verfügung zu stellen.

Aus der Druckschrift DE 10 2004 034 816 B4 ist eine Vorrichtung zur Auflage des Kopfes einer liegenden Person mit mehreren Huborganen versehen. An einem unteren zum Körper der Person weisenden Rand der Vorrichtung ist eine flexible Auflage angeordnet, in der eine Mehrzahl an über die gesamte Breite der Auflage verteilt angeordneten Druck- bzw. Temperatursensoren zur Ermittlung der Körperlage vorgesehen ist. Eine solche Vorrichtung kann lediglich die Körperlage der auf der Kopfunterlage ruhenden Person feststellen, nicht jedoch die Position des Kopfes auf der Kopfunterlage.

Die Druckschrift US 200/094 750 A1 zeigt eine Kopfunterlage zum Abstellen von Schnarchen, wobei die Kopfunterlage einen Vibrator aufweist, der abhängig von einem von Mikrophonen detektierten Schnarchen vibriert. Es sind Druckdetektionsschalter verteilt zuoberst der Kopfunterlage vorgesehen, um eine Lageänderung einer auf der Kopfunterlage ruhenden Person zu detektieren. Bei einer erkannten Lageänderung wird der Vibrator für eine bestimmte Zeit deaktiviert.

Die Druckschrift US 2004/177449 offenbart ein anpassbares Matratzen- und Kissensystem, bei dem abhängig von einer Belastung einer Sensormatte auf der Matratze die Konturen der Matratze und des Kissens angepasst werden. Die Sensormatte ist separat ausgebildet und erstreckt sich unter das Kissen.

Aus der Druckschrift US 2003/0221261 A1 ist eine Matratze mit Verformelementen zum bereichsweisen Anpassen ihrer Kontur bekannt, um einen gleichmäßig geringen Druck auf eine darauf liegende Personen auszuüben. Insbesondere werden die Verformelemente so angesteuert, dass eine Wirbelsäule der darauf liegenden Person geradlinig verläuft.

Die Druckschrift DE 91 00 663 U1 offenbart eine Vorrichtung zum Reduzieren des Schnarchens eines Schläfers, dessen Kopf während des Schlafens auf einem Kissen ruht, das ein Abrollgebilde aufweist, das sich im Kissenbereich in Verlängerung der Längsmittellinie des Körpers des Schläfers erstreckt und diesen zum seitlichen Abrollen zwingt, wobei das wulstartig ausgebildete Abrollgebilde von seinem dem Körper zugewandten Ende her und zum oben gelegenen Randbereich hin abgerundet ausgebildet ist und aus einem auffedernd nachgiebigen Material besteht.

Es ist Aufgabe der vorliegenden Erfindung, eine Kopfunterlage zur Verfügung zu stellen, bei der in verbesserter Weise die Position des Kopfes einer auf der Kopfunterlage ruhenden Person festgestellt werden kann. Eine weitere Aufgabe der vorliegenden Erfindung besteht darin, der auf der Kopfunterlage ruhenden Person einen verbesserten Komfort zur Verfügung zu stellen.

### Offenbarung der Erfindung

Diese Aufgabe wird durch die Kopfunterlage gemäß Anspruch 1 sowie durch das System zum Abstellen von Schnarchen gemäß dem nebengeordneten Anspruch gelöst.

Weitere vorteilhafte Ausgestaltungen der vorliegenden Erfindung sind in den abhängigen Ansprüchen angegeben.

Gemäß einem ersten Aspekt ist eine Kopfunterlage zum Beenden eines Schnarchens einer schlafenden Person vorgesehen, wobei die Kopfunterlage eine Kopfauflagefläche zum Auflegen des Kopfes der schlafenden Person aufweist. Die Kopfunterlage umfasst:
- eine aktive Lage mit einer Anordnung von zueinander benachbarten Verformelementen zum bereichsweisen Einstellen einer Höhe der Kopfunterlage;
- eine Sensorlage, die zwischen der Kopfauflagefläche und der aktiven Lage angeordnet ist, um eine Position des Kopfes auf der Kopfauflagefläche zu detektieren.

Eine Idee der obigen Kopfunterlage besteht darin, zur Erfassung der Position eines Kopfes einer auf der Kopfunterlage ruhenden Person eine Sensorlage zwischen einer Kopfauflagefläche der Kopfunterlage und einer aktiven Lage, die Verformelemente aufweist, vorzusehen, wobei die Sensorlage mehrere separate Sensorbereiche aufweist, so dass eine Position des Kopfes einer auf der Kopfunterlage ruhenden Person auf der Kopfauflagefläche bestimmt werden kann. Eine solche Anordnung ist einfach zu realisieren und insbesondere kann auf Sensoren zur Druckmessung in als Kammern ausgebildeten Verformelementen verzichtet werden.

Weiterhin kann eine Versteifungseinrichtung vorgesehen sein, um einer Auslenkung der aktiven Lage in Richtung einer Gewichtskraft des auf der Kopfauflagefläche aufliegenden Kopfes entgegen zu wirken.

Um bei vorgegebener Auflösung der Sensorlage eine verbesserte Positionserkennung des Kopfes durchführen zu können, ist weiterhin eine Versteifungseinrichtung zur Versteifung der aktiven Lage vorgesehen, die bei Aufliegen eines Kopfes auf der Kopfunterlage die Ausrichtung der einzelnen Verformelemente zueinander beibehält. Insbesondere soll die Versteifungseinrichtung bewirken, dass sich die Verformelemente an der Position, an der der Kopf auf der Kopfunterlage aufliegt, nicht in der dem Kopf gegenüberliegenden Seite der aktiven Lage auswölben, da hierdurch die Sensorlage ebenfalls verformt würde und dadurch unter Umständen keine zuverlässige bzw. genaue Detektion der Position des Kopfes mehr möglich wäre. Zwar könnte die Detektion der Position des Kopfes durch eine erhöhte Auflösung der Sensorlage verbessert werden, dies ist jedoch aufwändig und es kann durch die Versteifungseinrichtung zum Versteifen der aktiven Lage erreicht werden, bei gleich bleibender Genauigkeit der Erkennung der Position des Kopfes auf der Kopfunterlage eine Sensorlage mit relativ geringerer Auflösung zu verwenden.

Gemäß einer Ausführungsform kann die Versteifungseinrichtung eine Stützlage umfassen, die auf der der Sensorlage gegenüber liegenden Seite der aktiven Lage angeordnet ist, um die aktive Lage zu stützen.

Es kann vorgesehen sein, dass die aktive Lage mit der Stützlage, insbesondere durch Verkleben oder Vernähen, verbunden ist wobei zusätzlich oder alternativ die aktive Lage mit der Sensorlage, insbesondere durch Verkleben oder Vernähen, verbunden ist

Gemäß einer Ausführungsform kann die Stützlage zwischen einer Basislage und der aktiven Lage aufgenommen sein, wobei die Basislage eine geringere Steifheit aufweist als die Stützlage.

Weiterhin kann zwischen der Kopfauflagefläche und der Sensorlage eine Komfortlage angeordnet sein, wobei die Basislage und die Komfortlage zusammen die Stützlage, die aktive Lage und die Sensorlage vollständig umgeben.

Insbesondere kann die aktive Lage eine Anzahl erster Verformelemente aufweisen, die entlang einer Querrichtung in einer Reihe zueinander benachbart angeordnet sind, und ein oder mehrere zweite Verformelemente aufweisen, die sich in einer senkrecht zur Querrichtung verlaufenden Längsrichtung versetzt an die Reihe der in Querrichtung benachbart zueinander angeordneten ersten Verformelemente anschließen.

Die Verformelemente der aktiven Lage können mit einem Medium befüllbare Kammern aufweisen, wobei das Medium über eine Leitung durch eine extern der Kopfunterlage vorgesehene Pumpe bereitgestellt wird, wobei an bzw. in der Leitung eine Schalldämpfungseinrichtung angeordnet ist, um Schwingungen in dem Medium zu dämpfen. Weiterhin kann die Sensorlage Sensorbereiche mit einem oder mehreren resistiven, kapazitiven Drucksensoren, einem oder mehreren auf Druck Kontaktelementen oder einen oder mehreren Temperatursensoren aufweisen, wobei Sensorbereiche so angeordnet sind, dass die Position des Kopfes durch Identifizieren eines oder mehrerer der Sensorbereiche, die durch den Kopf beaufschlagt sind, ermittelbar ist.

Gemäß einer Ausführungsform kann vorgesehen sein, dass die Sensorlage einen Positionsensor umfasst, um durch ein kapazitives, induktives oder resistives Meßverfahren eine Angabe über eine Position des auf der Kopfauflagefläche aufliegenden Kopfes zu ermitteln.

Gemäß einem weiteren Aspekt ist ein System vorgesehen, umfassend:
- die obige Kopfunterlage;
- ein Steuergerät, um die Verformelemente der aktiven Lage abhängig von einer über die Sensorlage detektierten Position des Kopfes auf der Kopfauflagefläche anzusteuern, um die Lage und/oder Ausrichtung des Kopfes zu verändern.

Weiterhin kann das Steuergerät ausgebildet sein, um ein Schnarchen der auf der Kopfunterlage ruhenden Person zu erkennen und bei Erkennen des Schnarchens die Verformelemente zum Ändern der Lage und/oder Ausrichtung des Kopfes anzusteuern.

### Kurzbeschreibung der Zeichnungen

Bevorzugte Ausführungsformen der vorliegenden Erfindung werden nachfolgend anhand der beigefügten Zeichnungen näher erläutert. Es zeigen:
Figur 1 eine Darstellung eines Gesamtsystems zum Betreiben einer Kopfunterlage zum Abstellen von Schnarchen;
Figur 2 eine Querschnittsdarstellung des Lagenaufbaus der Kopfunterlage; und
Figur 3 eine schematische Ausschnittsdarstellung einer beispielhaften Sensorlage zur Verwendung in der Kopfunterlage der Figur 1.

### Beschreibung von Ausführungsformen

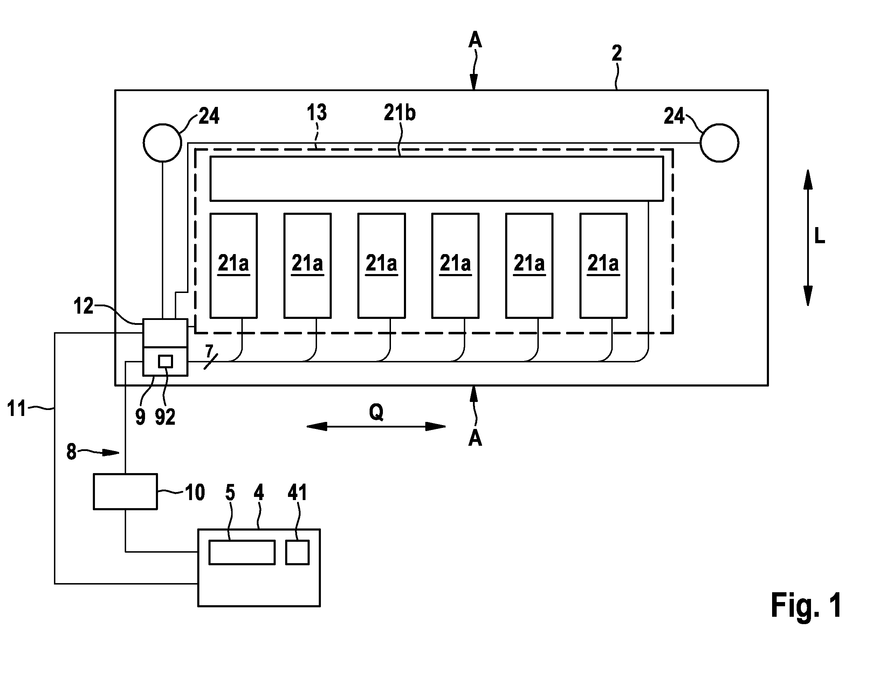

Figur 1 zeigt eine schematische Darstellung eines Systems 1 zum Abstellen eines Schnarchens. Das System 1 umfasst eine Kopfunterlage 2, die als aktives Kissen für eine schlafende Person oder als aktive Unterlage für den Kopf einer schlafenden Person dienen kann. Weiterhin steht die Kopfunterlage 2 mit einem Steuergerät 4 in Verbindung, das die mit der Kopfunterlage 2 auszuführenden Funktionen steuert. In einer alternativen Ausführungsform kann das Steuergerät 4 auch in der Kopfunterlage integriert sein.

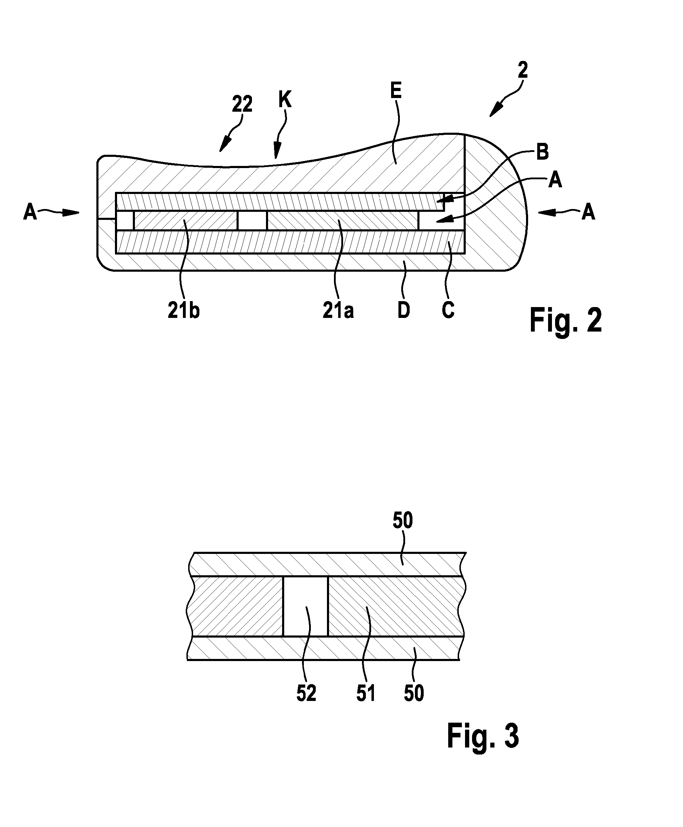

Die Kopfunterlage 2 weist eine im Wesentlichen rechteckige Form mit einer Querrichtung Q und einer Längsrichtung L auf. Die Kopfunterlage 2 soll so in einem Bett angeordnet sein, dass die Querrichtung Q der Breitenrichtung des Betts bzw. der Liegefläche und die Längsrichtung L der Längenrichtung des Betts bzw. der Liegefläche entspricht. Wie weiter in Verbindung mit der Seitenansicht der Kopfunterlage 2 in Figur 2 dargestellt, ist der Querschnitt der Kopfunterlage 2 bezüglich der Querrichtung Q mit variierender Höhe in der Höhenrichtung vorgesehen. Insbesondere weist die Kopfunterlage 2 eine Mulde 22 auf, um die Kopflage einer schlafenden Position bezüglich der Längsrichtung L zu definieren. Dies ist vorteilhaft, da dadurch eine gezielte Verkippbewegung des Kopfes in Längsrichtung L vereinfacht wird.

Die Kopfunterlage 2 weist wie in Figur 2 dargestellt eine aktive Lage A auf, die eine Anzahl von nebeneinander angeordneten (d. h. in Quer- und/oder Längsrichtung) Verformelementen 21 enthält. Die Verformelemente 21 können in geeigneter Weise angesteuert werden, um sich zu verformen, insbesondere um an ihrer jeweiligen Position in der Kopfunterlage 2 diese zu erhöhen oder abzusenken (in Bezug auf eine Richtung senkrecht zur Querrichtung Q und senkrecht zur Längsrichtung L).

Die Verformelemente 21 können, wie im dargestellten Ausführungsbeispiel, als gasbefüllbare Kammern aus flexiblem Material ausgebildet sein, insbesondere als Luftkammern. Die Kammern können mit einem Fluid, beispielsweise einem Gas, wie z. B. Luft, oder einer Flüssigkeit, beispielsweise einem Gel oder Wasser, befüllt werden. Die Menge an Fluid, das in die Kammern eingebracht wird, bestimmt die Verformung der Verformelemente 21 in der Kopfunterlage 2, insbesondere deren Dicke, so dass die Höhe der Kopfunterlage 2 an der Position des betreffenden Verformelements 21 selektiv variierbar ist.

Ein Reservoir von in die Kammern einzubringendem Fluid kann innerhalb der Kopfunterlage 2 oder davon separat vorgesehen sein. Im vorliegenden Ausführungsbeispiel ist das Steuergerät 4 extern der Kopfunterlage 2 vorgesehen und umfasst eine Pumpe für das Medium, mit dem die Kammern befüllt werden sollen, insbesondere eine Luftpumpe 5. Die Pumpe bzw. die Luftpumpe 5 kann als Membranpumpe ausgebildet sein und alternativ auch extern des Steuergeräts 4 angeordnet sein.

Die Luftpumpe 5 saugt die Umgebungsluft an und stellt diese unter einem erhöhten Druck bereit. Alternativ kann anstelle oder zusätzlich zur Luftpumpe 5 auch ein Druckluftbehälter vorgesehen sein, der außerhalb der Schlafenszeiten befüllt oder ausgetauscht werden kann, um Pumpgeräusche der Luftpumpe 5 während der Schlafenszeiten zu vermeiden.

Die Luftpumpe 5 ist über eine Luftleitung 8 mit der Kopfunterlage 2 verbunden. Da sich die Betriebsgeräusche der Pumpe 5 über die Luftleitung 8 in die Kopfunterlage 2 übertragen können, kann in der Luftleitung 8 zur Kopfunterlage 2 ein Schalldämpfungselement 10 angeordnet sein, das eine Schallübertragung durch die Luft in der Luftleitung 8 zur Kopfunterlage 2 dämpft bzw. unterbindet. Das Schalldämpfungselement 10 kann dazu ein Volumen aufweisen, durch das die in die Kammern zu transportierende Luft geführt wird. Das Volumen kann ein Hohlvolumen sein oder ein schalldämpfendes Material enthalten.

Anstelle von mit einem Medium befüllbaren Kammern können die Verformelemente 21 der aktiven Lage A auch als elektromechanische Aktuatoren oder dergleichen ausgeführt sein, solange diese eine selektive Höhenänderung eines Bereichs der Kopfunterlage 2 bewirken können.

Die Anzahl und Anordnung der Verformelemente 21 der aktiven Lage ist so gewählt, dass ein Verkippen bzw. Verdrehen eines auf der Kopfunterlage 2 aufliegenden Kopfes bezüglich mindestens einer Richtung, vorzugsweise der Querrichtung Q, durchgeführt werden kann. Dazu ist eine Reihe von gleichartigen ersten Verformelementen 21 a in der Querrichtung Q zueinander benachbart angeordnet. Die ersten Verformelemente 21 a können bezüglich der Höhenrichtung der Kopfunterlage 2 einen rechteckigen Querschnitt mit einer kürzeren Seite in Richtung der Querrichtung Q aufweisen. Auch eine mehreckige, runde, ovale oder wabenförmige Ausgestaltung der ersten Verformelemente 21 a kann vorgesehen werden.

Durch selektives Verformen der ersten Verformelemente 21 a kann eine Dreh- bzw. Verkippbewegung des aufliegenden Kopfes in der Querrichtung Q erfolgen. Dies wird insbesondere dadurch erreicht, dass zwei benachbarte erste Verformelemente 21 a, über die sich die Auflagefläche des aufliegenden Kopfes erstreckt, unterschiedlich verformt werden. Im Falle von befüllbaren Kammern als Verformelemente 21 kann dies dadurch erreicht werden, dass eine Kammer der zwei benachbarten ersten Verformelemente 21 a befüllt und die entsprechend andere Kammer der zwei benachbarten ersten Verformelemente 21 a entleert wird. Wenn sich die Auflagefläche des Kopfes über mehr als zwei Verformelemente 21 erstreckt, können selbstverständlich auch entsprechende Verformungen von mehr als zwei Verformelementen 21 vorgesehen werden, um das entsprechende Verkippen des Kopfes zu erreichen.

Um ein aktives Verkippen des Kopfes in der zur Querrichtung Q senkrechten Längsrichtung L zu erreichen, können ein oder mehrere zweite Verformelemente 21 b vorgesehen sein, die sich in Längsrichtung L an die in Querrichtung Q angeordnete Reihe von ersten Verformelementen 21 a anschließen und im Wesentlichen die gleiche Breite wie die Reihe der ersten Verformelemente 21 a einnehmen. Im gezeigten Ausführungsbeispiel erstreckt sich das zweite Verformelement 21 b über die gesamte Breite der Anordnung der ersten Verformelemente 21 a.

Es sind vielfältige Varianten der Anordnung der Verformelemente 21 denkbar, diese sollten jedoch so zueinander angeordnet sein, dass durch ein entsprechendes Verstellen eines oder mehrerer der Verformelemente 21 die Höhe der Kopfunterlage 2 in dem Bereich, in dem diese angeordnet sind, bzw. der Höhenunterschied zwischen den betreffenden Verformelementen 21 eingestellt bzw. verändert werden kann. Insbesondere soll mit der Anordnung der Verformelemente 21 erreicht werden, dass bei Aufliegen des Kopfes in einem aktiven Bereich der Kopfunterlage 2, in dem die Verformelemente 21 angeordnet sind, die jeweiligen Höhen von zumindest den unter der Auflagefläche des Kopfes angeordneten Verformelementen 21 so geändert werden können, dass eine Verkippung des Kopfes zumindest in der Querrichtung Q, vorzugsweise aber auch in der Längsrichtung L, erfolgen kann.

Im obigen Beispiel kann ein Verkippen dadurch erreicht werden, dass in dem Bereich, in dem der Kopf auf den Verformelementen 21 aufliegt, die Verformelemente 21 so angesteuert werden, dass die Höhe der Kopfunterlage 2 zunimmt, während in der Richtung, in die der Kopf verkippt werden soll, das entsprechende Verformelement 21 so angesteuert wird, dass die Kopfunterlage 2 dort eine geringere Höhe annimmt. Somit wirkt auf den Kopf ein Kippmoment, dessen Höhe im Wesentlichen von dem Höhenunterschied abhängt, dem der Kopf durch die unterschiedliche Einstellung der Höhen der benachbarten Verformelemente 21 ausgesetzt ist.

Im obigen Ausführungsbeispiel kann ein Verkippen des Kopfes in Längsrichtung L durch entsprechendes Verstellen des zweiten Verformelements 21 b erreicht werden. Zur Unterstützung der Verkippbewegung in Längsrichtung L können zusätzlich die in Querrichtung Q angeordneten Verformelemente 21 dazu entgegengesetzt angesteuert werden, so dass sie eine gleichförmige, über die gesamte Breite der Anordnung in Querrichtung Q entgegen der Höhenänderung des zweiten Verformelements 21 b wirkende Höhenänderung, also entweder eine Erhöhung oder eine Verringerung der Höhe der Kopfunterlage 2, bewirken.

Die Kopfunterlage 2 ist weiterhin mit einem oder mehreren Mikrofonen 24 versehen, durch die es möglich ist, ein Schnarchgeräusch der auf der Kopfunterlage 2 schlafenden Person aufzunehmen. Um das Schnarchgeräusch zuverlässig zu detektieren, sind vorzugsweise mindestens zwei Mikrofone 24 an den Enden der Kopfunterlage 2 in Querrichtung Q angeordnet. Dadurch kann auch das Schnarchgeräusch einer auf der Seite liegenden schlafenden Person zuverlässig detektiert werden.

Anstelle durch die Mikrofone 24 kann das Schnarchgeräusch auch durch einen in der Kopfunterlage 2 angeordneten Drucksensor (nicht gezeigt) oder durch einen externen Schwingungsensor (nicht gezeigt) detektiert werden.

Weiterhin kann die Kopfunterlage 2 mit einer sensitiven Folie, wie beispielsweise eine Folie mit resistiven, kapazitiven oder piezoresistiven Messeigenschaften, versehen sein, um die durch Schnarchgeräusche verursachten Vibrationen zu detektieren. Alternativ dazu kann auch ein Bettgestell, auf dem die Kopfunterlage 2 aufliegt, mit entsprechender Sensorik versehen sein, um durch ein Schnarchen bedingte Vibrationen zu erfassen.

Die Ansteuerung der Verformelemente 21 erfolgt durch das externe Steuergerät 4. Das Steuergerät 4 steht in Signalverbindung mit einer Stelleinheit 9, in der Solenoidventile 92 bzw. andere geeignete Ventile zum Steuern einer Luftzufuhr oder Luftabfuhr, wie z.B. Trommel- oder Reed-Ventile, für jedes der Verformelemente 21 a, 21 b vorgesehen sind. Die Solenoidventile 92 werden elektrisch von dem Steuergerät 4 über eine Signalverbindung 11 angesteuert und sind in der Lage, die über die Luftleitung 8 gelieferte Druckluft an die Kammern der Verformelemente 21 a, 21 b weiterzuleiten. Allgemein können die Solenoidventile 92 die Luftzufuhr zu und die Luftabfuhr von den einzelnen Kammern steuern. Mit anderen Worten ermöglichen es die Solenoidventile 92, jede der Kammern einzeln so anzusteuern, dass diese mit Druckluft befüllt werden können oder die darin befindliche Luft abgelassen werden kann.

Das Steuergerät 4 steht weiterhin über eine (optionale) Aufbereitungseinheit 12 mit einem Sensorfeld 13 einer Sensorlage B und den Mikrofonen 24 in Signalverbindung, so dass das Steuergerät 4 sowohl ein von den Mikrofonen 24 bereitgestelltes elektrisches Mikrofonsignal als auch von dem Sensorfeld 13 bereitgestellte Sensorsignale zur Verfügung gestellt werden können. Die Aufbereitungseinheit 12 dient dazu, die Anzahl der Signalleitungen, die zu dem Steuergerät 4 geführt werden, zu reduzieren. Erfasst mindestens eines der Mikrofone 24 ein Schnarchgeräusch, so wird ein entsprechendes Mikrofonsignal durch das Steuergerät 4 ausgewertet, um ein Schnarchen zu erkennen bzw. festzustellen, ob es sich bei dem erfassten Geräusch um ein Schnarchgeräusch handelt oder nicht. Dazu kann in einem Mikrocontroller 41 des Steuergeräts 4 ein geeigneter Klangerkennungsalgorithmus implementiert sein, der im Prinzip eine Funktion eines Spracherkennungsalgorithmus umfassen kann.

Bei Erkennen eines Schnarchgeräuschs steuert das Steuergerät 4 die Verformelemente 21 in geeigneter Weise an. Dazu erfasst das Steuergerät 4 über das Sensorfeld 13 die Position des Kopfes auf der Auflagefläche der Kopfunterlage 2 und ermittelt diejenigen Verformelemente 21 a, 21 b, mit denen eine Lageänderung des Kopfes bewirkt werden kann. Diese Verformelemente 21 a, 21 b werden dann gemäß einem vorgegebenen Algorithmus durch Ansteuern der Solenoidventile 92 angesteuert, um eine Höhenänderung der betreffenden Verformelemente 21 a, 21 b unter dem aufliegenden Kopf zu erreichen. Selbstverständlich können jedoch auch elektromechanische Aktuatoren als Verformelemente 21 mithilfe von elektrischen Zuleitungen durch das Steuergerät 4 angesteuert werden.

Um eine gezielte Lageänderung des Kopfes auf der Kopfunterlage 2 vornehmen zu können, ist es notwendig, die Position des Kopfes auf der Kopfunterlage 2 mit einer Genauigkeit bzw. Ortsauflösung zu erkennen, die ausreicht, um die zur Lageänderung des Kopfes notwendigen Verformelemente 21 a, 21 b identifizieren zu können. Dazu ist, wie in der Querschnittsansicht der Figur 2 dargestellt, die Sensorlage B im Inneren der Kopfunterlage 2 vorgesehen, die zwischen der aktiven Lage A und der Kopfauflagefläche K des Kopfes vorgesehen ist und im Wesentlichen die aktive Lage A flächig überdeckt. Insbesondere kann die Sensorlage B unmittelbar auf der aktiven Lage A aufliegen.

Die Sensorlage B kann die aktive Lage A ganz oder teilweise überdecken. Die Sensorlage B kann einzelne Sensorelemente aufweisen, um eine Druckbeaufschlagung durch Aufliegen des Kopfes auf einem Bereich der Kopfauflagefläche K, der dem betreffenden Sensorelement zugeordnet ist bzw. unmittelbar über diesem liegt, zu erkennen. Insbesondere kann jedem der Verformelemente 21, 21 a, 21 b ein oder mehrere Sensorelemente zugeordnet sein, die sich vorzugsweise an einer der Kopfauflagefläche K nächstliegenden Position des betreffenden Verformelements 21, 21 a, 21 b befinden.

Die Sensorlage B sollte also so bezüglich der aktiven Lage A angeordnet sein, dass die Sensorlage B mit durch das Aufliegen des Kopfes der schlafenden Person bewirkten Druck beaufschlagbar ist. Insbesondere sollte die Sensorlage B so im Inneren der Kopfunterlage angeordnet sein, dass die aktive Lage A als Widerlager für eine Druckbeaufschlagung dienen kann.

Die aktive Lage A kann weiterhin nicht aktiv verformbare Elemente aufweisen, über die sich die Sensorlage B ebenfalls erstreckt. Insbesondere das zweite Verformelement 21 b kann durch ein nicht aktiv verformbares Stützelement, insbesondere ein Nackenstützelement ersetzt sein.

Die Sensorlage B weist mehrere Sensorbereiche auf, in denen jeweils ein Aufliegen eines Kopfes detektiert werden kann. Die Messprinzipien in den Sensorbereichen können auf kapazitiven oder resistiven Druckmessungen basieren, wie sie aus dem Stand der Technik bekannt sind.

Wie in Figur 3 dargestellt, kann eine bevorzugte Ausführungsform der Sensorlage B für jeden Sensorbereich zwei parallel voneinander beabstandete Flächenelemente 50 aufweisen, die an den zueinander gerichteten Oberflächen leitend ausgebildet sind. Beispielsweise sind die Flächenelemente 50 aus einer elastischen Folie oder Schicht ausgebildet, die jeweils an den zueinander gerichteten Oberflächen mit einer metallischen Schicht, wie z. B. einer Silberschicht, versehen sind. Zwischen den Flächenelementen 50 ist ein elektrisch isolierendes, elastisch verformbares Trennmaterial 51, wie z. B. Schaumstoff oder dergleichen, angeordnet. Das Trennmaterial 51 weist für jeden Sensorbereich eine oder mehrere Ausnehmungen 52 auf, an denen die beiden leitfähigen zueinander gerichteten Oberflächen bei entsprechender Druckbeaufschlagung senkrecht zur Flächenrichtung miteinander in elektrischen Kontakt gelangen können. Diese elektrische Kontaktierung kann für jeden Sensorbereich separat detektiert werden und in dem Steuergerät 4 ausgewertet werden. Auf diese Weise ist ein einfacher Aufbau einer Sensorlage B möglich, durch die das Einwirken einer Kraft in besonders einfacher Weise festgestellt werden kann.

Anstelle der Detektion einer Kontaktierung der zwei einander gegenüberliegenden Flächenelemente 50 kann auch eine Änderung der zwischen den Flächenelementen 50 bestehenden Kapazität detektiert werden, wenn durch eine Druckbeaufschlagung das elastisch verformbare Trennmaterial 51 gestaucht wird und dadurch sich der Abstand zwischen den Flächenelementen 50 verringert.

Als Alternative zur Ausführung der Sensorlage B mit Drucksensoren kann ein Vorsehen eines Sensorfelds mit Temperatursensoren, beispielsweise resistiven Temperatursensoren, in den Sensorbereichen vorgesehen sein. Bei einer Änderung des elektrischen Widerstands eines Sensorelements in einem Sensorbereich, durch die eine Temperaturerhöhung erkannt werden kann, kann das Aufliegen eines Kopfes detektiert werden. Die Detektion der Lage des Kopfes auf der Kopfunterlage 2 mithilfe von Temperatursensoren ist insbesondere vorteilhaft, da hierdurch eine wünschenswerte Tiefpassfilterung der Angabe der Position des Kopfes aufgrund der thermischen Trägheit implizit erreicht wird.

Allgemein kann die Kopflage mithilfe eines Positionssensors ermittelt werden, mit dem es möglich ist, unter Nutzung eines geeigneten Messprinzips die Position eines auf der Kopfauflagefläche K aufliegenden Kopfes zu bestimmen. Als Messprinzipien kommen kapazitive, resistive und induktive Messverfahren zum Erkennen einer Kraft-/Druckbeaufschlagung, Temperaturbeaufschlagung und/oder einer Änderung von elektromagnetischen Eigenschaften an einem Sensorbereich der Sensorlage B in Frage.

Bei Untersuchungen wurde festgestellt, dass die Position des Kopfes auf der Kopfauflagefläche K nicht mit einer hinreichenden Genauigkeit bzw. Auflösung festgestellt werden kann, wenn aufgrund des Eigengewichts des Kopfes die aktive Lage A bezüglich ihrer Form im unbelasteten Zustand gebogen oder in sonstiger Weise verformt wird, d. h. sich die Anordnung der einzelnen Verformelemente 21 a, 21 b bezüglich ihrer Anordnungsrichtung im unbelasteten Zustand ändert. Insbesondere tritt häufig der Fall auf, dass durch Belastung des Kopfes die aktive Lage A durchgebogen wird und sich in die Unterlage, auf der die Kopfunterlage 2 aufliegt, eindrückt. Dies führt dazu, dass der durch die Sensorlage B erkannte Auflagebereich des Kopfes zu groß wird, um die tatsächliche Position des Kopfes eindeutig feststellen zu können. Aus diesem Grund ist es sinnvoll, eine Versteifung der aktiven Lage A zu erreichen.

Dies kann beispielsweise, wie in der Querschnittsdarstellung der Figur 2 gezeigt, durch das Vorsehen einer Stützlage C erreicht werden, die bezüglich der aktiven Lage A der Sensorlage B und der Kopfauflagefläche K gegenüber liegt. Eine Druckbeaufschlagung seitens der Kopfauflagefläche K kann dann nicht zu einem nennenswerten Durchbiegen der aktiven Lage A führen, da diese durch die Stützlage C gestützt wird. Die Stützlage C dient dazu, die aktive Lage A zu versteifen und gegen eine Auslenkung aus ihrer Anordnungsrichtung im unbelasteten Zustand zu stützen.

Die Abmessungen der Stützlage C in der Längs- und Querrichtung entsprechen im Wesentlichen den Abmessungen der aktiven Lage A. Um einen direkten Kontakt der auf der Kopfunterlage 2 ruhenden Person mit der Stützlage C zu vermeiden, kann eine Basislage D vorgesehen sein, die im Wesentlichen eine der aktiven Lage A gegenüber liegende Seite der Stützlage C überdeckt und die gemeinsam mit einer auf der der aktiven Lage A gegenüber liegenden Seite der Sensorlage B aufgebrachten Komfortlage E die Stützlage C, die aktive Lage A und die Sensorlage B umgibt.

Die Basislage D und die Komfortlage E sind aus einem für Kissen üblichen Material, z. B. einem Polstermaterial wie Schaumstoff oder dergleichen, ausgebildet, um je nach Wunsch Weichheit und Komfort zu bieten.

Zur Gewährleistung eines sicheren Halts der Stützlage C und der aktiven Lage A kann die Basislage hochgezogene Ränder aufweisen, so dass eine Wannenform ausgebildet wird. Die Wannenform hält zumindest die Stützlage C gegen ein Verrutschen in seitlicher Richtung (in Querrichtung Q bzw. in Längsrichtung L).

Die Stützlage C ist vorzugsweise aus einem Material ausgebildet, das eine höhere Steifigkeit aufweist als das Material der Basislage D. Beispielsweise können als Materialien für die Stützlage C ein Kunststoffmaterial, Metall und/oder ein Naturfasermaterial in Betracht gezogen werden, die keine oder keine nennenswerte Durchbiegung bei einer Belastung der Kopfauflagefläche K mit dem Gewicht eines Kopfes erfahren. Insbesondere sollte das Material so gewählt werden, dass eine etwaige Durchbiegung der Stützlage C nicht zu einer Detektion einer Auflagefläche des Kopfes führt, die größer ist als die tatsächliche Auflagefläche des Kopfes auf der Kopfauflagefläche K.

Um ein Verschieben der aktiven Lage A auf der Stützlage C zu vermeiden, ist die aktive Lage A auf der Stützlage C befestigt. Beispielsweise kann die aktive Lage A auf die Stützlage C aufgeklebt sein. In einer alternativen Ausführungsform kann die aktive Lage A auch auf die Stützlage C aufgenäht sein. Dazu kann die aktive Lage A Verformelemente 21 a, 21 b mit befüllbaren Kammern aufweisen und die Stützlage C aus einem Naturfasermaterial, wie beispielsweise Jute, ausgebildet sein. Das Aufnähen kann dann durch Nähte zwischen den Kammern erfolgen.

In vergleichbarer Weise kann auch die Sensorlage B mit der aktiven Lage A und der Stützlage C durch Verkleben und/oder Vernähen verbunden werden, um eine Verschiebung der Sensorlage B auf der aktiven Lage A zu vermeiden. Dies ist insbesondere deshalb wichtig, da eine Zuordnung der Sensorbereiche der Sensorlage B zu den darunter befindlichen Verformelementen 21 a, 21 b eindeutig sein muss, um die zu stellenden Verformelemente 21 a, 21 b identifizieren zu können.

## Patentansprüche

1. Kopfunterlage (2) zum Beenden eines Schnarchens einer schlafenden Person, wobei die Kopfunterlage (2) eine Kopfauflagefläche (K) zum Auflegen des Kopfes der schlafenden Person aufweist, umfassend:
- eine aktive Lage (A) mit einer Anordnung von zueinander benachbarten Verformelementen (21, 21 a, 21 b) zum bereichsweisen Einstellen einer Höhe der Kopfunterlage (2);
- eine Sensorlage (B), die im Inneren der Kopfunterlage (2) zwischen der Kopfauflagefläche (K) und der aktiven Lage angeordnet ist, um eine Position des Kopfes auf der Kopfauflagefläche (K) zu detektieren,
wobei die Sensorlage (B) Sensorbereiche aufweist, um die Position des Kopfes auf der Kopfunterlage (2) durch Identifizieren eines oder mehrerer der Sensorbereiche, die durch den Kopf der schlafenden Person beaufschlagt sind, zu ermitteln, so dass die für eine Lageänderung des Kopfes notwendigen Verformelemente (21, 21 a, 21 b) identifizierbar sind.

2. Kopfunterlage (2) nach Anspruch 1, wobei eine Versteifungseinrichtung vorgesehen ist, um einer Auslenkung der aktiven Lage (A) in Richtung einer Gewichtskraft des auf der Kopfauflagefläche (K) aufliegenden Kopfes entgegen zu wirken.

3. Kopfunterlage (2) nach Anspruch 2, wobei die Versteifungseinrichtung eine Stützlage (C) umfasst, die auf der der Sensorlage (B) gegenüber liegenden Seite der aktiven Lage (A) angeordnet ist, um die aktive Lage (A) zu stützen.

4. Kopfunterlage (2) nach Anspruch 3, wobei die aktive Lage (A) mit der Stützlage (C), insbesondere durch Verkleben oder Vernähen, verbunden ist und/oder wobei die aktive Lage (A) mit der Sensorlage (B), insbesondere durch Verkleben oder Vernähen, verbunden ist

5. Kopfunterlage (2) nach Anspruch 3 oder 4, wobei die Stützlage (C) zwischen einer Basislage (D) und der aktiven Lage (A) aufgenommen ist, wobei die Basislage (D) eine geringere Steifheit aufweist als die Stützlage (C).

6. Kopfunterlage (2) nach Anspruch 5, wobei zwischen der Kopfauflagefläche (K) und der Sensorlage (B) eine Komfortlage (E) angeordnet ist, wobei die Basislage (D) und die Komfortlage (E) zusammen die Stützlage (C), die aktive Lage (A) und die Sensorlage (B) vollständig umgeben.

7. Kopfunterlage (2) nach einem der Ansprüche 1 bis 6, wobei die Verformelemente (21, 21 a, 21 b) der aktiven Lage (A) mit einem Medium befüllbare Kammern aufweisen, wobei das Medium durch eine extern der Kopfunterlage (2) vorgesehenen Pumpe über eine Leitung (8) bereitgestellt wird, wobei in der Leitung (8) eine Schalldämpfungseinrichtung (10) angeordnet ist, um Schwingungen in dem Medium zu dämpfen.

8. Kopfunterlage (2) nach einem der Ansprüche 1 bis 7, wobei die Sensorlage (B) Sensorbereiche mit einem oder mehreren resistiven, kapazitiven Drucksensoren, einem oder mehreren auf Druck empfindlichen Kontaktelementen oder einem oder mehreren Temperatursensoren aufweist, wobei Sensorbereiche so angeordnet sind, dass die Position des Kopfes durch Identifizieren eines oder mehrerer der Sensorbereiche, die durch den Kopf beaufschlagt sind, ermittelbar ist.

9. System (1) umfassend:
- eine Kopfunterlage (2) nach einem der Ansprüche 1 bis 8;
- ein Steuergerät (4), um die Verformelemente (21, 21 a, 21 b) der aktiven Lage (A) abhängig von einer über die Sensorlage (B) detektierten Position des Kopfes auf der Kopfauflagefläche (K) anzusteuern, um die Lage und/oder Ausrichtung des Kopfes zu verändern.

10. System (1) nach Anspruch 9, wobei das Steuergerät (4) ausgebildet ist, um ein Schnarchen der auf der Kopfunterlage ruhenden Person zu erkennen und bei Erkennen des Schnarchens die Verformelemente (21, 21 a, 21 b) zum Ändern der Lage und/oder Ausrichtung des Kopfes anzusteuern.

## Claims

1. A head support (2) for stopping a snoring of a sleeping individual, wherein the head support (2) has a head resting surface (K) for the head of a sleeping individual to rest on, comprising:
- an active layer (A) with an arrangement of neighboring deforming elements (21,. 21a, 21b) for controlling the height of the head support (2) section by section;
- a sensing layer (B) which is arranged in the interior of the head support (2) between the head resting surface (K) and the active layer to detect a position of the head resting on the head resting surface (K),
wherein the sensing layer (B) has sensing sections to determine the position of the head on the head support (2) by identifying one or more of the sensing sections on which the head rests on, so that the deforming elements (21, 21a, 21b) necessary for changing the position of the head are identifiable.

2. The head support (2) according to claim 1, wherein a stiffening element is provided to counteract a deflection of the active layer (A) in the direction of a weight force of the head resting on the head resting surface (K).

3. The head support (2) according to claim 2, wherein the stiffening element comprises a backing layer (C) which is arranged on the side of the active layer (A) opposing the sensing layer (B) to back the active layer (A).

4. The head support (2) according to claim 3, wherein the active layer (A) is attached to the backing layer (C) particularly by adhering or by sewing, and/or wherein the active layer (A) is attached to the sensing layer (B) particularly by adhering or by sewing.

5. The head support (2) according to claim 3 or 4, wherein the backing layer (C) is sandwiched between a base layer (D) and the active layer (A), wherein the base layer (D) has a lower stiffness than the backing layer (C).

6. The head support (2) according to claim 5, wherein a comfort layer (E) is sandwiched between the head resting surface (K) and the sensing layer (B) wherein the base layer (D) and the comfort layer (E) together fully enclose the backing layer (C), the active layer (A) and the sensing layer (B).

7. The head support (2) according to any of the claims 1 to 6, wherein the deforming elements (21, 21a, 21b) of the active layer (A) comprise chambers fillable with a medium, wherein the medium is supplied via a conduct (8) by means of a pump provided external the head support (2) wherein a sound-dampening device (10) is arranged on or in the conduct (8) to dampen vibrations in the medium.

8. The head support (2) according to any of the claims 1 to 7, wherein the sensing layer (B) comprise sensing sections with one or more resistive, capacitive pressure sensors, one or more pressure-sensitive contact elements or one or more temperature sensors wherein the sensing sections are arranged so that the position of the head is detectable by identifying one or more of the sensing sections on which the head rests/lies on.

9. A system comprising:
- the head support according to any of the claims 1 to 8,
- a control unit to actuate the deforming elements of the active layer depending on a position of the head of the head resting surface detected by means of the sensing layer to change the position and/or the orientation of the head.

10. The system according to claim 9 wherein the control unit (4) is configured to detect the snoring of an individual resting on the head support (2) and to actuate the deforming elements (21, 21a, 21 b) to change the position and/or the orientation of the head when the snoring has been detected.

## Revendications

1. Support de tête (2) destiné à mettre fin au ronflement d'une personne endormie, dans lequel le support de tête (2) présente une surface d'appuie-tête (K) pour la pose de la tête de la personne endormie, comprenant:
- une couche active (A) avec un agencement d'éléments de déformation (21, 21 a, 21 b) voisins l'un de l'autre pour le réglage local d'une hauteur du support de tête (2);
- une couche de détection (B), qui est agencée à l'intérieur du support de tête (2) entre la surface d'appuie-tête (K) et la couche active, afin de détecter une position de la tête sur la surface d'appuie-tête (K),
dans lequel la couche de détection (B) présente des zones de détection, destinées à déterminer la position de la tête sur le support de tête (2) par identification d'une ou de plusieurs zones de détection, qui sont occupées par la tête de la personne endormie, de telle manière que les éléments de déformation (21, 21 a; 21b) nécessaires pour un changement de position de la tête puissent être identifiés.

2. Support de tête (2) selon la revendication 1, dans lequel il est prévu un système de renforcement destiné à s'opposer à un déplacement de la couche active (A) dans la direction d'une force due au poids de la tête posée sur la surface d'appuie-tête (K).

3. Support de tête (2) selon la revendication 2, dans lequel le système de renforcement comprend une couche de soutien (C), qui est disposée sur le côté de la couche active (A) situé à l'opposé de la couche de détection (B), afin de soutenir la couche active (A).

4. Support de tête (2) selon la revendication 3, dans lequel la couche active (A) est assemblée à la couche de soutien (C) en particulier par collage ou couture, et/ou dans lequel la couche active (A) est assemblée à la couche de détection (B) en particulier par collage ou par couture.

5. Support de tête (2) selon la revendication 3 ou 4, dans lequel la couche de soutien (C) est disposée entre une couche de base (D) et la couche active (A), dans lequel la couche de base (D) présente une rigidité moindre que la couche de soutien (C).

6. Support de tête (2) selon la revendication 5, dans lequel une couche de confort (E) est agencée entre la surface d'appuie-tête (K) et la couche de détection (B), dans lequel la couche de base (D) et la couche de confort (E) entourent ensemble entièrement la couche de soutien (C), la couche active (A) et la couche de détection (B).

7. Support de tête (2) selon l'une quelconque des revendications 1 à 6, dans lequel les éléments de déformation (21, 21a, 21b) de la couche active (A) présentent des chambres pouvant être remplies avec un fluide, dans lequel le fluide est fourni via une conduite (8) par une pompe prévue à l'extérieur du support de tête (2), dans lequel un système d'insonorisation (10) est agencé dans la conduite (8) pour amortir les vibrations dans le fluide.

8. Support de tête (2) selon l'une quelconque des revendications 1 à 7, dans lequel la couche de détection (B) présente des zones de détection avec un ou plusieurs détecteurs de pression résistifs, capacitifs, un ou plusieurs éléments de contact sensibles à la pression ou un ou plusieurs détecteurs de température, dans lequel des zones de détection sont agencées de telle manière que la position de la tête puisse être déterminée par identification d'une ou de plusieurs zones de détection, qui sont activées par la tête.

9. Système (1) comprenant:
- un support de tête (2) selon l'une quelconque des revendications 1 à 8;
- un appareil de commande (4), pour commander les éléments de déformation (21, 21 a, 21b) de la couche active (A) en fonction d'une position de la tête sur la surface d'appuie-tête (K) détectée par la couche de détection (B), afin de modifier la position et/ou l'orientation de la tête.

10. Système (1) selon la revendication 9, dans lequel l'appareil de commande (4) est configuré de façon à reconnaître un ronflement de la personne reposant sur le support de tête et, lors de la reconnaissance du ronflement, de commander les éléments de déformation (21, 21a, 21b) de façon à modifier la position et/ou l'orientation de la tête.
